# EUROPEAN PATENT SPECIFICATION

(11) **EP 3 504 324 B1**
(45) Date of publication and mention of the grant of the patent: **04.05.2022**
(21) Application number: 17764626.2
(22) Date of filing: 24.08.2017
(51) Int. Cl.: C12N 5/079

(54) **DIFFERENTIATION OF PLURIPOTENT STEM CELLS INTO CORNEAL CELLS**
DIFFERENZIERUNG VON PLURIPOTENTEN STAMMZELLEN IN HORNHAUTZELLEN
DIFFÉRENCIATION DE CELLULES SOUCHES PLURIPOTENTES EN CELLULES CORNÉENNES

(30) Priority: 24.08.2016 FI 20165627
(43) Date of publication of application: 03.07.2019
(73) Proprietor: StemSight Oy, 33880 Lempäälä (FI)
(72) Inventor: ILMARINEN, Tanja, 33014 Tampereen Yliopisto (FI); HONGISTO, Heidi, 33014 Tampereen Yliopisto (FI); SKOTTMAN, Heli, 33014 Tampereen Yliopisto (FI); MIKHAILOVA, Alexandra, 33014 Tampereen Yliopisto (FI)
(74) Representative: Papula Oy
(86) International application number: PCT/FI2017/050595
(87) International publication number: WO 2018/037161

(56) References cited:
- EP-A1- 2 828 380
- EP-A1- 3 031 906
- WO-A1-2012/073238
- ALEXANDRA MIKHAILOVA ET AL: "Small-Molecule Induction Promotes Corneal Epithelial Cell Differentiation from Human Induced Pluripotent Stem Cells", STEM CELL REPORTS, vol. 2, no. 2, 1 February 2014 (2014-02-01), pages 219-231, XP055108416, ISSN: 2213-6711, DOI: 10.1016/j.stemcr.2013.12.014
- MIKHAILOVA ALEXANDRA ET AL: "Human pluripotent stem cell-derived limbal epithelial stem cells on bioengineered matrices for corneal reconstruction", EXPERIMENTAL EYE RESEARCH, ACADEMIC PRESS LTD, LONDON, vol. 146, 30 November 2015 (2015-11-30), pages 26-34, XP029562830, ISSN: 0014-4835, DOI: 10.1016/J.EXER.2015.11.021
- KATHRYN L. MCCABE ET AL: "Efficient Generation of Human Embryonic Stem Cell-Derived Corneal Endothelial Cells by Directed Differentiation", PLOS ONE, vol. 10, no. 12, 21 December 2015 (2015-12-21), page e0145266, XP055413958, DOI: 10.1371/journal.pone.0145266

## Description

### FIELD OF THE INVENTION

The present description relates to differentiation of stem cells into eye precursor cells and further into differentiated eye cells, such as corneal epithelial cells. Accordingly, here is provided means and methods contributing to fast and effective induction, maturation and differentiation of stem cells towards corneal lineage cells.

### BACKGROUND OF THE INVENTION

Pluripotent stem cells provide great opportunities for corneal reconstruction by cell-based therapies. The cornea is located at the front surface of the eye and is multi-layered, transparent and avascular in structure. The main functions of the cornea are to protect the eyeball and its contents, while allowing accurate focusing of light to produce a sharp image on the retina. The cornea consists of three cellular layers, namely epithelium, stroma and endothelium, separated by two acellular layers - Bowman's layer, and Descemet membrane. As the outermost layer, corneal epithelium is exposed to the external environment and thereby needs to be rapidly regenerating and stratified. Similarly to the epidermis, lens and conjunctival epithelium, corneal epithelium originates from the surface ectoderm. However, detailed developmental mechanisms and signaling routes remain unknown.

Methods for corneal differentiation of pluripotent stem cells are known in the art. Many of the methods are slow or provide only modest differentiation efficiencies. For example, Hayashi et al., 2012 reported corneal cell differentiation of human induced pluripotent stem cells on mouse-derived feeder cells taking 12-16 weeks and resulting in a differentiation efficiency of less than 15% based on the expression of CK12, whereas Yoshida et al., 2011 managed to produce corneal precursor cells by differentiation of mouse induced pluripotent stem cells through cultivation on mouse-derived feeder cells by a method which took about 60 days.

Ahmad et al., 2007 used medium conditioned by limbal fibroblasts for culturing human embryonic stem cells previously maintained on a feeder layer of mouse embryonic fibroblasts. Said culturing resulted in the loss of pluripotency and differentiation into epithelial like cells. They reported a differentiation efficiency of 50% on day 5 and 10% on day 21 as measured by expression of proteins CK3/12. However, use of a medium which requires donated limbal cells can be considered problematic. Moreover, there is significant biological variation between batches of limbal cells.

The methods mentioned above, as well as most other current methods of maintaining or differentiating human pluripotent stem cells, utilize a culture environment produced by mouse-derived feeder cells, such as mouse embryonic fibroblast (MEF) cells. Owing to their exposure to animal-derived materials, clinical use of such stem cells is problematic.

In some other methods, mouse-derived feeder cells have been replaced with feeder cells of human origin, such as human foreskin fibroblasts. For example, EP 2 828 380 and Mikhailova et al. (Stem Cell Reports, 2014, Vol. 2, pp 219-231) disclose an efficient method of differentiating human pluripotent stem cells previously maintained on human foreskin fibroblasts into epithelial precursor cells. The differentiation method disclosed is a two-step method which comprises an induction step, preferably carried out on a suspension culture, wherein the pluripotent stem cells are cultured in the presence of a TGF-beta inhibitor, a Wnt inhibitor, and a fibroblast growth factor, thereby producing eye precursor cells. Said eye precursor cells are then differentiated, in an adherent culture, into corneal epithelial precursor cells in the presence of epidermal growth factor, hydrocortisone, insulin, isoproterenol, and tri-iodo-thyronine. Optionally, said corneal epithelial precursor cells may be maturated further into mature corneal epithelial cells or into corneal stratified epithelium.

New feeder-free techniques for maintaining pluripotent stem cells have become available. For example, Rodin et al. (Nature Communications, 2014, 5:3195) reported a feeder-free, xeno-free culture system for human pluripotent cells, which is based on culturing the cells on a substrate coated with recombinant Laminin-521 and E-cadherin in defined, xeno-free culture medium. According to flow cytometric analyses made by the present inventors, stem cells in the feeder-free culture system show greater positivity to pluripotency markers as compared with stem cells cultured on feeder cells. Owing to their greater pluripotency, it is advantageous to use stem cells maintained in a feeder-free cell culture system for producing differentiated cells. Also, culturing stem cells is more cost effective, less laborious, and more standardized in the absence of feeder cells.

Unexpectedly, existing corneal differentiation methods may not work equally well with pluripotent stem cells obtained from a feeder-free culture than with pluripotent cells maintained on feeder cells. For example, the above-mentioned differentiation method disclosed in EP 2828380 and Mikhailova et al. (ibid.) cannot be used to produce cells of the corneal lineage from pluripotent cells maintained on feeder-free conditions very successfully.

EP 3031906 discloses a production method of a cell aggregate comprising an anterior eye segment tissue (lens and cornea) or a partial structure thereof, or a precursor tissue thereof. In the method, an aggregate of pluripotent stem cells is cultured in suspension in the presence of a bone morphogenic factor signal transduction pathway activating substance, such as BMP-4, to induce neural retinal self-organization inside the aggregate. The period necessary for the induction of the neural retinal tissue may vary depending on the culture conditions but if human pluripotent stem cells are used, the induction of the neural retina takes at least 8 days. Upon continued suspension culture in the presence of the bone morphogenic factor signal transduction pathway activating substance, the self-organized neural retinal tissue drives anterior eye differentiation on the surface of the cell aggregate. For use e.g. in corneal transplantation, the self-organized corneal tissue must be isolated from the remaining anterior eye segment tissue and the cell aggregate.

Publication WO 2012/073238 describe a method of generating a population of corneal epithelial cells, comprising culturing human pluripotent stem cells in corneal fibroblast-conditioned medium on a solid surface comprising an extracellular matrix component.

Publication Mikhalilova et al. Experimenta Eye Research 146 (2016) 26-34 discloses a method for differentiating human pluripotent stem cells into limbal epithelial stem cells by culturing the cells in suspension and then in the presence of a TGFbeta inhibitor, a wnt inhibitor and bFGF.

McCabe et al. PLOS one, 10(12) December 21, 2015 discloses a method for the efficient generation of corneal endothelial cells from human embryonic stem cells using a feeder free culture.

Thus, there still is need for improved methods for differentiating stem cells towards cells of the corneal lineage, which cells may be used to treat and study corneal epithelium conditions, diseases, and pathologies, as well as used for toxicological studies and drug development, especially in cases where the stem cells are obtained from cell cultures lacking feeder cells. Further, avoidance of xeno-derived or undefined components is also an important aim.

### BRIEF DESCRIPTION OF THE INVENTION

In one aspect, the present invention provides a method of producing differentiated eye cells selected from the group consisting of corneal epithelial precursor cells, corneal epithelial cells and stratified corneal epithelium, the method comprising
a) culturing pluripotent stem cells in the absence of feeder cells;
b) culturing said cells in a cell culture medium comprising a TGF-beta inhibitor and a fibroblast growth factor (FGF) followed by culturing said cells in a cell culture medium comprising bone morphogenetic protein 4 (BMP-4) thereby producing eye precursor cells;
c) culturing on a substrate coated at least with collagen IV and laminin said eye precursor cells in a cell culture medium comprising one or more supplements selected from the group consisting of epidermal growth factor (EGF), hydrocortisone, insulin, isoproterenol, and tri-iodo-thyronine in the absence of a TGF-beta inhibitor, FGF, or BMP-4, thereby producing corneal epithelial precursor cells; and
d) optionally, maturating said corneal epithelial precursor cells further into mature corneal epithelial cells or into corneal stratified epithelium.

In some embodiments, the induction phase of the present method is carried out in a suspension culture. Accordingly, the present invention also provides a method of producing differentiated eye cells selected from the group consisting of corneal epithelial precursor cells, corneal epithelial cells and stratified corneal epithelium, the method comprising
a) culturing pluripotent stem cells in the absence of feeder cells, and forming embryoid bodies from said pluripotent stem cells;
b) culturing said embryoid bodies in a cell culture medium comprising a TGF-beta inhibitor and a fibroblast growth factor (FGF) followed by culturing said embryoid bodies in a cell culture medium comprising bone morphogenetic protein 4 (BMP-4) thereby producing eye precursor cells;
c) culturing on a substrate coated at least with collagen IV and laminin said eye precursor cells in a cell culture medium compris-ing one or more supplements selected from the group consisting of epidermal growth factor (EGF), hydrocortisone, insulin, isoproterenol, and tri-iodo-thyronine
   in the absence of a TGF-beta inhibitor, FGF, or BMP-4, thereby producing corneal epithelial precursor cells; and
d) optionally, maturating said corneal epithelial precursor cells further into mature corneal epithelial cells or into corneal stratified epithelium.

The cell culture medium used in b) and/or in c) does not contain any Wnt-inhibitors.

Other objectives, aspects, embodiments, details and advantages of the present invention will become apparent from the following figures, detailed description, experimental part, and dependent claims.

### BRIEF DESCRIPTION OF THE DRAWINGS

In the following, the invention will be described in greater detail by means of preferred embodiments with reference to the attached drawings, in which
Figure 1 exemplifies structures of two commercial small molecular TGF-β1-inhibitors (D 4476 and SB 505124).
Figure 2 shows immunofluorescence stainings of cells after 11 days of differentiation. The cells start to show epithelial morphology and expression of p63alpha and PAX6. Scale bars 200 µm. BF, bright field.

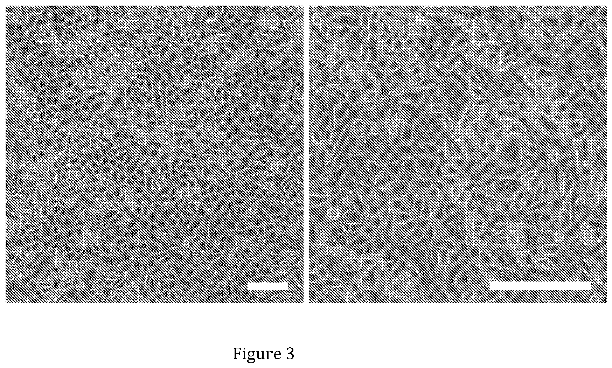

Figure 3 illustrates the morphology of cells after 30 days of differentiation. Scale bars 200 µm.

### DETAILED DESCRIPTION OF THE INVENTION

In some aspects, the present invention provides a method of producing differentiated corneal cells including corneal epithelial precursor cells, mature corneal epithelial cells, and even corneal stratified epithelium, through inducing pluripotent stem cells first into eye precursor cells and then differentiating the eye precursor cells into corneal cells. Characteristics of these cell types are well known in the art, for example as disclosed in Mikhailova et al., Exp Eye Res. 2015 146:26-34. As used herein, the terms "precursor" and "progenitor" may be used interchangeably unless otherwise indicated.

### Starting material

In the present method, undifferentiated pluripotent stem cells obtained from a feeder-free culture are used as a starting material for producing differentiated eye cells.

As used herein, the term "pluripotent stem cell" refers to any stem cell having the potential to differentiate into all cell types of a human or animal body, not including extra-embryonic tissues. These stem cells include both embryonic stem cells (ESCs) and induced pluripotent cells (iPSCs). Hence, the cells suitable for the method of the present invention include stem cells selected from iPSCs and ESCs. Human pluripotent stem cells (hPSCs) are preferred and they include human iPSCs (hiPSCs) and human ESCs (hESCs).

ESCs are of great therapeutic interest because they are capable of indefinite proliferation in culture and are thus capable of supplying cells and tissues for replacement of failing or defective human tissue. Producing eye precursor cells from human embryonic stem cells may meet ethical challenges. According to one embodiment, human embryonic stem cells may be used with the proviso that the method itself or any related acts do not include destruction of human embryos.

Induced pluripotent stem cells, commonly abbreviated as iPS cells or iPSCs are a type of pluripotent stem cell artificially derived from a non-pluripotent cell, typically an adult somatic cell, by inducing a forced expression of specific genes by means and methods well known in the art. An advantage of using iPS cells is that no embryonic cells have to be used at all, so ethical concerns can be avoided. A further advantage is that production of patient-specific cells without immunorejection problems is enabled by employing iPSC technology. Therefore, according to another embodiment of the present invention, use of iPS cells is preferred. For clinical use, hiPS cells are preferred.

Induced pluripotent stem cells are similar to natural pluripotent stem cells, such as embryonic stem cells, in many aspects, such as the expression of certain stem cell genes and proteins, chromatin methylation patterns, doubling time, embryoid body formation, teratoma formation, viable chimera formation, and potency and differentiability, but the full extent of their relation to natural pluripotent stem cells is still being assessed. Induced pluripotent cells are typically made from adult skin cells, blood cells, stomach, or liver, although other alternatives may be possible. A man skilled in the art is familiar with research and therapy potential of iPS cells, i.e. from publication of Bilic and Izpiua Belmonte (2012).

Pluripotent stem cells are difficult to maintain in cell culture because they tend to follow their natural cell fate and differentiate spontaneously. To prevent unwanted differentiation, pluripotent stem cells are typically cultured on feeder cells. Animal-derived feeder cells, such as mouse embryonic fibroblasts (MEFs), are widely used as feeder cells even for human pluripotent cells. To replace animal-derived materials, human-derived feeder cells, such as human foreskin feeder cells, have also been employed for culturing human pluripotent stem cells. However, in some cases, these cells have proven unsuitable for maintenance of pluripotent stem cells.

To overcome drawbacks associated with the use of feeder cells, new feeder-free cell culturing methods have been developed. Accordingly, the term "feeder-free culture" or any linguistic variation thereof, refers to a culture of pluripotent stem cells in the absence of any feeder cells.

Use of feeder cells can be omitted, for example, by replacing them with an appropriate substrate coating as is well known in the art. Suitable coating materials include, but are not limited to, human or animal, natural extracted or recombinant extracellular matrix (ECM) proteins such as laminins, collagens, vitronectin, fibronectin, nidogens, proteoglycans, and E-cadherin, as well as isoforms, fragments, and peptide sequences thereof. Non-limiting examples of said ECM protein isoforms include different isoforms of laminin, such as Laminin-511, -521, -322, -411 etc. Non-limiting examples of said fragments include E8 fragments of human laminin isoforms, whereas one non-limiting example of said peptide sequences is an Arg-Gly-Asp (RGD) sequence of vitronectin. Said ECM proteins, isoforms, fragments, or peptide sequences may also be fused to other proteins, N-cadherin domain fused to an IgG-Fc domain being one non-limiting example of such fusion proteins. Alternative or additional suitable coating materials include, but are not limited to, ECM or basement membrane extracts from different tissue or cell types of human or animal origin, such as mouse embryonic fibroblast, human fibroblasts, mesenchymal stem cells, and tumours. Further suitable coating materials include natural or synthetic biomaterials and hybrids thereof, either alone or as functionalised with ECM proteins or sequences thereof or other chemical or physical surface modifications. Non-limiting examples of such biomaterials include PMEDSAH (poly[2-(methacryloyloxy)ethyl-dimethyl-(3-sulfopropyl)ammonium hydroxide) and collagen-grafted Mixed Cellulose Esters membrane (MCE-COL). Non-limiting examples of further suitable coating materials include commercial products such as Corning^{®} Synthemax^{®} surface, Corning^{®} PureCoat^{™}, CELLstart^{™}, Matrigel^{™}, or Geltrex^{®}. Any of the above-mentioned proteins, isoforms, fragments, peptide sequences, fusion proteins, extracts, biomaterials or commercial products may be used either alone or in any appropriate combinations or mixtures to replace feeder cells as is well known in the art. Means and methods for obtaining, selecting, and using suitable coating materials for replacing feeder cells are readily available in the art.

Preferred extracellular matrix proteins include laminins, heterotrimeric glycoproteins that contain a α-chain, a β-chain, and a γ-chain, found in five, four, and three genetic variants, respectively. The laminin molecules are named according to their chain composition. Thus, as used herein, the term "laminin-521" refers to a laminin containing α5, β2, and γ1 chains, whereas the term "laminin-511" refers to a laminin containing α5, β1, and γ1 chains. Preferred laminins include recombinant human laminin-521, recombinant human laminin-511, and fragment E8 of recombinant human laminin-511.

While human pluripotent stem cells grow as colonies on feeder cells, they grow in a feeder-free culture on laminin-521 as a monolayer allowing more effective expansion. Moreover, according to flow cytometric analyses, human pluripotent stem cells cultured in a feeder-free culture system, show greater positivity to pluripotency markers as compared with cells cultured on feeder cells.

Indeed, human pluripotent stem cells obtained from a culture containing feeder cells or from a feeder-free culture appear to be different. As demonstrated in the experimental part, corneal differentiation method disclosed in EP 2828380 perform well with human pluripotent stem cells obtained from a culture comprising feeder cells, but it does not perform equally well with pluripotent cells obtained from a feeder-free culture. The present method, however, provides excellent differentiation of human pluripotent stem cells obtained from a feeder-free culture into corneal epithelial precursor cells.

### Induction phase

In the induction phase of the present method, pluripotent stem cells obtained from a feeder-free culture are induced towards surface ectoderm and eye precursor cells. As used herein, the term "eye precursor cell" refers broadly to any cell lineage of the eye induced from pluripotent stem cells and characterized by down-regulation of the pluripotency marker OCT-4 (also known as POU5F1) and up-regulation of PAX6, a gene indicating differentiation into eye specific cell lineages.

The induction phase may be carried out either in a suspension culture or in an adherent culture. If the induction phase is to be carried out in adherent culture, it may be advantageous to use substrates coated with materials such as extracellular matrix (ECM) proteins or combinations thereof as generally known in the art. Preferred ECM proteins include, but are not limited to laminins, collagens, vitronectin, fibronectin, nidogens, and proteoglycans or peptide sequences thereof. Moreover, any coatings suitable for replacing feeder cells may be used to enable the induction phase to be carried out in adherent culture.

In some embodiments, it is preferable to carry out the induction phase in a suspension culture. In such embodiments, a first step of the induction phase comprises forming embryoid bodies from pluripotent stem cells obtained from a feeder-free culture. As used herein, the term "embryoid body" (EB) refers to a three-dimensional cell aggregate. Formation of embryoid bodies can be achieved by various aggregation-promoting methods well known in the art.

As used herein, the term "aggregation-promoting method" refers to any method capable of promoting formation of embryoid bodies from pluripotent stem cells by physical or chemical means.

Formation of embryoid bodies can be achieved, for example, by employing a physical aggregation-promoting method, wherein pluripotent stem cells are cultured in a suspension culture in the presence of non-attachment promoting cell culture surfaces, such as Corning Corning^{®} Costar^{®} Ultra-Low attachment surfaces, or in the presence of one or more agents that prevent cell attachment.

Further non-limiting examples of suitable physical methods of promoting aggregation to achieve EB formation include hanging drop cultures. In such methods, a suspension containing pluripotent stem cells is plated on a lid of a petri dish in regular arrays, inverted lid is then placed over the bottom of a petri dish filled with an appropriate liquid, such as PBS, to prevent the drops from drying out. Eventually, the stem cells fall to the bottom of the hanging drops, and aggregate into a single EB per drop. Also commercial products based on the hanging drop method, such as Perfecta3D^{®} Hanging Drop Plates (3D Biomatrix), may be employed.

Suitable physical aggregation-promoting methods include also methods which are based on multiwell and microfabrication technologies and employ, for example, low-adherence 96-well plates or microwell arrays to induce formation of EBs with a controlled size. Non-limiting examples of such plates or arrays include microwell-patterned poly(dimethylsiloxane) (PDMS) molds with microwells, agarose hydrogel micro-well arrays, as well as commercial Aggre-Well^{™} (STEMCELL Technologies) microarray plates.

Also forced aggregation by centrifugation or rotation, or by other physical environments such as microgravity environments may be employed as a physical aggregation-promoting method for obtaining EB formation.

Further suitable aggregation-promoting methods include culturing of cells in presence of agents which aid cell aggregation. Non-limiting examples of such agents include macromolecular crowders, which force cells to closer contact, such as galactose derivatives (e.g. carrageenan), glucose derivatives (e.g. dextran sulfate), polyethylene glycol, and Ficoll^{®}.

Also physical aggregation-promoting methods based on encapsulation or entrapment of pluripotent stem cells in hydrogels, such as methylcellulose, fibrin, hyaluronic acid, dextran, alginate, or agarose, thereby generating individually separated EBs in a semi-solid suspension media, may be used.

Any suitable physical aggregation-promoting method may be used in combination with one or more aggregation promoting chemical agents such as blebbistatin or Rho-associated kinase (ROCK) inhibitors or any other apoptosis inhibiting agents that enhance single cell survival and/or promote stem cell aggregation. Alternatively, cell aggregation may be promoted by chemical means only, for instance by using the chemical agents set forth above.

Conventionally, embryoid bodies may also be formed from pluripotent stem cells by manual separation of adherent colonies or regions of adherent colonies. However, in some embodiments of the present method, manual separation is not a feasible option for obtaining embryoid bodies because pluripotent cells cultured in feeder-free conditions do not spontaneously re-aggregate after manual dissociation to small aggregates or single cells. Addition of one or more aggregation-promoting agents, such as ROCK inhibitors or Blebbistatin, markedly diminish dissociation-induced apoptosis and enables formation of embryoid bodies after dissociation.

Time required for forming embryoid bodies may vary depending on different variables such as the method to be used. Typically the duration of this step is between about 1 hour and about 48 hours, more specifically between about 5 hours and about 24 hours, or overnight, i.e. about 18 hours. However, in some cases, the duration of this step may be even longer than 48 hours. In some embodiments, pluripotent stem cells are cultured in the presence of blebbistatin for about 1 day for the formation of embryoid bodies.

In a second step of the induction phase, either adherent cells or embryoid bodies obtained from the first step of the induction phase are subjected to an induction medium comprising active "induction supplements", i.e. a TGF-beta inhibitor and a fibroblast growth factor. These induction supplements were found to enhance induction of pluripotent stem cells towards eye precursor cells and improve their further differentiation efficiency into clinically valuable eye cells, such as corneal epithelial precursor cells, corneal epithelial cells, or stratified fully maturated corneal epithelium.

In some preferred embodiments, the amount of the TGF-beta inhibitor in the induction medium is from 1 µM to 100 µM, preferably from 1 to 30 µM, and/or the amount of fibroblast growth factor is from 1 ng/ml to about 1000 ng/ml, preferably about 2 ng/ml to about 100 ng/ml, and more preferably about 30 ng/ml to about 80 ng/ml.

The present induction medium may be considered to consist of or comprise a basal medium and the present induction supplements. However, further supplements common in the art may be applied. As used herein, the term "common cell culture supplements" refers to ingredients used in practically every cell culture medium including antibiotics, L-glutamine, and serum, serum albumin or a serum replacement, preferably a defined serum replacement.

On the other hand, in some embodiments, the induction medium does not contain ingredients other than the induction supplements, basal medium, antibiotics, L-glutamine, and a defined serum replacement.

In some more specific embodiments, a TGF-beta inhibitor of Formula I or II, and bFGF are used as the induction supplements. In some even more specific embodiments, the induction supplements are SB-505124, and bFGF. In some still even more specific embodiments, SB-505124 is used in a concentration of 10 µM and bFGF is used in a concentration of 50 ng/ml.

Any of the aforementioned embodiments may form a basis for additional or alternative embodiments, wherein the induction medium does not comprise any supplements generally known to be inductive for differentiation types other than differentiation towards eye lineages, such types as neural differentiation. Such generally known supplements include, but are not limited to, retinoic acid, ascorbic acid, brain-derived neurotrophic factor BDNF, and glial-derived neurotrophic factor GDNF. In some embodiments, the induction medium does not contain any Wnt inhibitors. Without being limited to any theory, omitting Wnt inhibitors may be advantageous owing to the possible toxicity of Wnt inhibitors or otherwise adverse effects thereof, such as adverse effects on cell survival as shown in Example 1.

Time used for inducing the adherent cells or the embryoid bodies with the induction supplements may vary depending on different variables such as the cell line, early differentiation status of the cells, and the specific supplements to be used and concentrations thereof. Typically the duration of this step varies from about 1 day to about 7 days (i.e. from about 22 hours to about 185 hours), more specifically from about 1 day to about 5 days (i.e. from about 22 hours to about 132 hours). In some embodiments, the embryoid bodies are cultured in the presence of induction supplements for about 1 day (i.e. about 22 to 26 hours).

In a third step of the induction phase, induction supplements are withdrawn and the adherent cells or embryoid bodies obtained from the second step of the induction phase are cultured in the presence of bone morphogenetic protein 4 (BMP-4) to drive the cells towards surface ectoderm and, concomitantly, to prevent differentiation towards neural lineages. Typical concentrations include 1 ng/ml to 1000 ng/ml, preferably about 10 ng/ml to 50 ng/ml, and more preferably 25 ng/ml.

Time used for inducing the adherent cells or embryoid bodies with BMP-4 may vary depending on different variables such as the concentration of BMP-4. Typically, the duration of this step varies from about 12 hours to about 5 days (i.e. from about 12 hours to about 132 hours), preferably from about 24 hours to about 4 days (i.e. from about 24 hours to about 105 hours), and more specifically 2 days (i.e. from about 43 hours to about 53 hours), and it may or may not involve replacing the culture medium with fresh medium. In some embodiments, the third step is carried out for about 2 days, preferably first for about 1 day in medium supplemented with BMP-4, preferably in an amount of 25 ng/ml, and then about 1 further day in fresh medium also supplemented with BMP-4, preferably in an amount of 25 ng/ml.

Overall duration of the induction phase may vary depending on different variables. Typically, the duration of the induction phase may vary from a couple of days to several days. A preferred time range is from about 2.5 days to about 18 days, i.e. from about 54 hours to about 475 hours. In some embodiments, preferred overall duration of the induction phase is from about 3 to about 7 days (i.e. from about 65 hours to about 185 hours, or from about 3 days to about 5 days (i.e. from about 65 hours to about 132 hours). More specifically, a preferred overall duration of the induction phase is 3 days. As used herein, the term "about" refers to a variation of about 10 percent of the value specified. Thus, the term "about three days" carries a variation from 65 to 79 hours, while the term "about seven days" carries a variation from 152 to 186 hours, for example. If shorter induction times are used, down-regulation of OCT4 and up-regulation of PAX6 may be weak leading to less efficient differentiation as determined by weak expression of precursor markers PAX6 and p63. Moreover, if longer induction times are used, more neural differentiation can be expected, because human pluripotent stem cells have a known tendency to differentiate towards neural lineages, especially in the presence of basic fibroblast growth factor.

### Aggregation-promoting agent

As used herein, the functional term "aggregation-promoting agent" refers to an agent capable of promoting formation of embryoid bodies. Non-limiting examples of aggregation-promoting agents include blebbistatin and Rho-associated kinase (ROCK) inhibitors disclosed in more detail below.

Blebbistatin is a cell-permeable, selective, and reversible inhibitor of nonmuscle myosin II. The name is derived from its ability to inhibit cell blebbing. The actin-myosin based cytoskeleton is a dynamic system essential for cell contraction, motility, and tissue organization. Actin-myosin motors consist of actin filaments and non-muscle myosin II heavy chains that slide along actin filaments, resulting in contraction. The process is triggered by the binding of myosin light chain, which is activated by phosphorylation through kinases, such as Rho-associated kinase (ROCK). Removal from ECM and epithelial cell contacts leaves actin-myosin free to contract, generating altered phenotypes, including excessive cell membrane blebbing and ultimately cell death. Disruption of actin-myosin contraction in individualized human ES cells dramatically improves cell survival and cloning efficiency. Actin-myosin contraction is a downstream target of ROCK regulation. Blebbistatin is readily available in the art.

ROCK inhibitors are cell-permeable, highly potent and selective inhibitors of Rho-associated coiled-coil forming protein serine/threonine kinase (ROCK) family of protein kinases. Non-limiting examples of ROCK inhibitors include chroman 1, Fasudil, Fasudil Hydrochloride, GSK269962A, GSK429286A, H-1152, H-1152 dihydrochloride, Hydroxyfasudil, Hydroxyfasudil hydrochloride, K-115, K-115 free base, LX7101, RKI-1447, ROCK inhibitor(azaindole 1), SAR407899, SAR407899 hydrochloride, SLx-2119, SR-3677, Thiazovivin, and Y-27632, all available e.g. from MedChem Express.

A preferred ROCK inhibitor is Y-27632 (dihydrochloridetrans-4-[(1R)-1-Aminoethyl]-N-4-pyridinylcyclohexanecarboxamide dihydrochloride), which inhibits both ROCK1 and ROCK2 by competing with ATP for binding to the catalytic site. Moreover, it is a potent inhibitor of pluripotent stem cell apoptosis (anoikis), permits survival of dissociated human pluripotent stem cells, improves embryoid body formation in forced-aggregation protocols, and increases the survival of cryopreserved single human ES cells after thawing.

Those skilled in the art can easily determine using various methods readily available in the art, whether or not a given agent has aggregation-promoting activity or not, and whether it is suitable for use in the present method. Non-limiting examples of such methods include visual evaluation of aggregate formation and cell viability assays.

### TGF-beta (TGF-β) inhibitor

As used herein, with "TGF-beta inhibitor" is referred functionally to a substance capable of inhibiting transforming growth factor β1.

Transforming growth factor β1 (TGF-β1) is a member of a large superfamily of pleiotropic cytokines that are involved in many biological activities, including growth, differentiation, migration, cell survival, and adhesion in diseased and normal states. Nearly 30 members have been identified in this superfamily. These are considered to fall into two major branches: TGFβ/Activin/Nodal and BMP/GDF (Bone Morphogenetic Protein/Growth and Differentiation Factor). They have very diverse and often complementary functions. Some are expressed only for short periods during embryonic development and/or only in restricted cell types (e.g. anti-Mullerian hormone, AMH, Inhibin) while others are widespread during embryogenesis and in adult tissues (e.g. TGFβ1 and BMP4). TGF-β1 is a potent regulator in the synthesis of the extracellular matrix (fibrotic factor) and plays a role in wound healing.

In chemical and structural terms, suitable TGF-beta inhibitory function may be found among proteins and small organic molecules. A man skilled in the art is aware of means for isolating proteins from biological matrixes or producing them i.e. by recombinant techniques.

Compounds exhibiting TGF-beta inhibitory activity may be found by screening. Preferably a TGF-beta inhibitor is an organic molecule having a relatively low molar mass, e.g. a small molecule having molar mass less than 800 g/mol, preferably less than 500 g/mol. As a general structure, Formula I, a suitable low molar mass TGF-inhibitor may be described as: 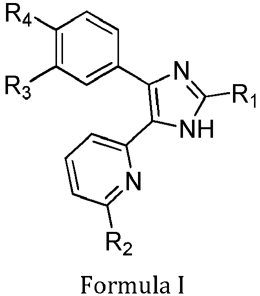 wherein R₁ represents a C₁-C₅ aliphatic alkyl group, carboxylic acid, amide, and R₂ represents a C₁-C₅ aliphatic alkyl, R₃ and R₄ represent aliphatic alkyls including heteroatoms, 0 or N, which may be linked together to form a 5- or 6 member heteroring.

A typical structure comprises a hetero ring having 2 oxygen atoms, when it can be referred to as a small molecule of general formula II: 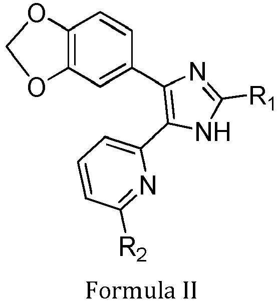 wherein, R₁ represents a C₁-C₅ aliphatic alkyl group, an aromatic carboxylic acid or amide, and R₂ represents a C₁-C₅ aliphatic alkyl.

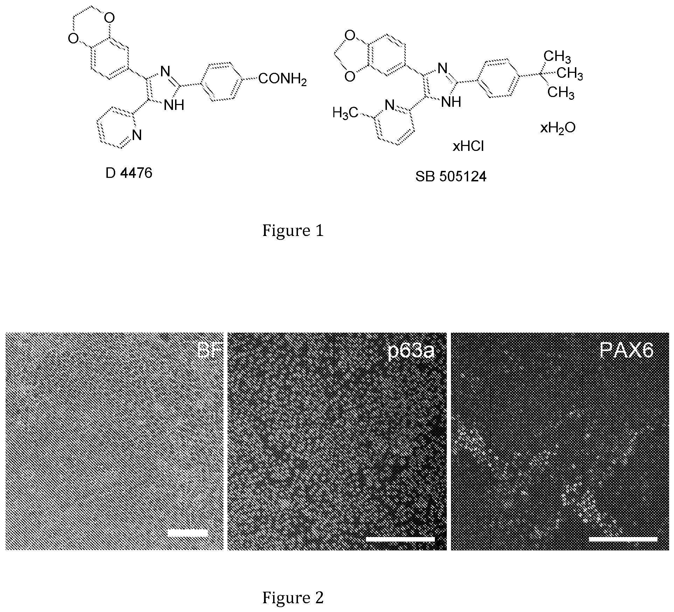

One example of such an TGF-b1-inhibitor is 4-[4-(1,3-Benzodioxol-5-yl)-5-(2-pyridinyl)-1H-imidazol-2-yl]-benzamide; 4-[4-(3,4-Methylenedioxyphenyl)-5-(2-pyridyl)-1H-imidazol-2-yl]-benzamide; 4-(5-benzol[1,3]dioxol-5-yl-4-pyridin-2-yl-1H-imidazol-2-yl)-benzamide hydrate, with the chemical formula in figure 1, which is commercially available from suppliers and marketed as a selective inhibitor of transforming growth factor-β type I receptor (ALK5), ALK4 and. Selectively inhibits signaling from TGF-β and activin; does not inhibit other ALK family members. Another example of TGF-inhibitors is 2-(5-benzo[1,3]dioxol-5-yl-2-tert-butyl-3H-imidazol-4-yl)-6-methylpyridine hydrochloride hydrate, the structure of which is given in figure 1 as well.

However, other small molecules exhibiting TGF-beta inhibitory activity or commercially marketed as TGF-inhibitors may be equally suitable in the context of the present invention. When selecting said TGF-beta inhibitor from substances obtainable by chemical synthesis or recombinant production, a defined medium can be provided. It also complies with requirements of xeno-free and serum-free conditions.

Those skilled in the art can easily determine, using various methods readily available in the art, whether or not a given agent has TGF-beta inhibiting activity or not, and whether it is suitable for use in the present method.

### Fibroblast growth factor

In the induction medium of the present invention, a fibroblast growth factor is required to contribute to the differentiation. Fibroblast growth factors, or FGFs, are a family of growth factors generally involved in angiogenesis, wound healing, and embryonic development. The FGFs are heparin-binding proteins and interactions with cell-surface-associated heparan sulfate proteoglycans have been shown to be essential for FGF signal transduction. FGFs are key players in the processes of proliferation and differentiation of wide variety of cells and tissues.

Fibroblast growth factors suitable for use in the present invention include fibroblast growth factors (FGFs) such as basic FGF (bFGF or FGF-2). While FGF is preferably used, other materials, such as certain synthetic small peptides (e.g. produced by recombinant DNA variants or mutants) designed to activate fibroblast growth factor receptors, may be used instead of FGF. Fibroblast growth factors may be included in the serum replacement used as basal medium or they may be added separately to the final cell culture medium according to the present invention.

### Differentiation phase

The above-described induction phase, preferably but not necessarily carried out in a suspension culture, is followed by a differentiation phase in adherent culture. In the latter phase, eye precursor cells produced in the induction phase are differentiated into corneal epithelial precursor cells. Optionally, the differentiation method may further comprise a maturation phase, also carried out in adherent culture, wherein the corneal epithelial precursor cells are maturated further into mature corneal epithelial cells and even stratified corneal epithelium.

In this context, the term "corneal epithelial precursor cells" refers to cells which are positive for, i.e. express, one or more corneal epithelial markers including, but not limited to, p63, CK15, ABCG2, TCF4, and BMI-1, which may be quantified for example with the help of immunofluorescent stainings. According to an embodiment, the corneal epithelial precursor cells expressing p63 represent at least 65%, preferably at least 75%, most preferably at least 90% of the total cell population. On the other hand, cultures of limbal stem cells wherein p63 positive cells represent even as little as only 3% of the cell population have been reported to provide therapeutic success rate of 78% (Rama et al. 2010, N Engl J Med, 363:147-55). Accordingly, in some embodiments, a population of corneal epithelial precursor cells may comprise as little as only 3% or more p63 positive cells, and still be a clinically relevant cell population and encompassed by the term "corneal epithelial precursor cells".

In practice, the differentiation phase and maturation phase are carried out successively in the same way; they only differ from each other in respect of timing, as explained in more detail below. Even the culture medium to be used in these stages is the same. Thus, the terms "differentiation medium" and "maturation medium" are interchangeable. The same applies to the terms "differentiation supplements" and "maturation supplements". If desired, a shift from a differentiation stage to a maturation stage may concern only a selected subpopulation of cells obtained from the differentiation stage.

Since the differentiation and maturation phases are to be performed in an adherent culture and the ability to attach to extracellular matrix (ECM) is considered to be important for epithelial cells, it is advantageous to use substrates, such as cell culture plates or bottles, coated with ECM proteins as generally known in the art. Preferred ECM proteins include collagen IV and laminin, preferably laminin-521 and/or laminin-511. More preferably, the cell culture substrate is coated with a mixture of collagen IV and laminin-521 and/or laminin-511, even more preferably with 5 µg/cm² of collagen IV and 0.75 µg/cm² of laminin-521. Other non-limiting examples of suitable coating materials include collagen I, vitronectin, fibronectin, nidogens, proteoglycans, or peptide sequences thereof, commercial attachment and culture substrates comprising the same, such as CELLstart^{™}, and basement membrane extracts, such as Matrigel^{™} or Geltrex^{®}. Moreover, any coatings suitable for replacing feeder cells may be used in differentiation and maturation phase. When xeno-free conditions are desired for human use, the substrate is to be coated with one or more ECM proteins of human or recombinant human origin. Means and methods for coating cell culture substrates are generally available in the art.

Typically, obtaining corneal epithelial precursor cells requires culturing eye precursor cells under the present corneal differentiation conditions for about 10 to about 35 days, preferably for about 25 days. In some preferred embodiments, corneal epithelial precursor cells are obtained by carrying out the induction phase for about 2.5 days to about 18 days followed by the corneal differentiation phase for about 20 to 26 days. The most pure p63-positive cell population can be obtained after a differentiation stage of this length. Shorter differentiation time yields more heterogeneous cell populations, while longer differentiation time results in terminal maturation towards corneal epithelial cells.

In some further embodiments, said corneal epithelial precursor cells may be maturated even further into mature corneal epithelial cells or stratified corneal epithelium, as demonstrated by a characteristic marker expression and morphology. Non-limiting examples of markers for maturated corneal epithelium include CK12 and CK3. Such further maturation may be obtained by continued cell culturing, typically for an additional 10 to 20 days, in the present corneal maturation conditions, which in practice correspond to the corneal differentiation conditions. This embodiment may be termed as a three-stage differentiation method.

A suitable culture medium for use in the differentiation and maturation stages may be, for instance, any available corneal medium such as CnT-30 which is commercially available from CELLnTECH, or any supplemental hormonal epithelial medium (SHEM) suitable for culturing corneal epithelial cells. In some other embodiments, the differentiation and maturation medium may be composed by adding ingredients such as one or more differentiation and maturation supplements selected from the group consisting of epidermal growth factor (EGF), hydrocortisone, insulin, isoproterenol and tri-iodo-thyronine, into any suitable basal medium. In some embodiments, the corneal differentiation and maturation medium does not contain ingredients other than said one or more differentiation and maturation supplements, basal medium, antibiotics, L-glutamine, and a defined serum replacement. In other words, in some embodiments, the corneal differentiation medium and maturation medium does not contain any of the following ingredients: a TGF-beta inhibitor, a fibroblast growth factor, and BMP-4, or any functionally equivalent agents. In some further embodiments, said corneal differentiation medium does not comprise a Wnt-inhibitor either.

Any of the aforementioned embodiments may form a basis for additional or alternative embodiments, wherein the differentiation and maturation medium does not comprise any supplements, which are generally known to cause differentiation towards cells types other than eye cells, such types as neural differentiation. Such generally known supplements include, but are not limited to, retinoic acid, ascorbic acid, brain-derived neurotrophic factor BDNF, and glial-derived neurotrophic factor GDNF.

Importantly, differentiation of pluripotent stem cells into corneal epithelial precursor cells and further maturation into corneal epithelial cells, or stratified corneal epithelium was significantly better when the cells were differentiated by carrying out the three step induction phase followed by the present corneal differentiation and maturation phase than when performing induction phase as disclosed in EP 2 828 380 or when performing longer or shorter induction phase. Improved results were obtained e.g. regarding attachment to combination coating with collagen IV and laminin-521 comparing to collagen IV alone, uniform corneal epithelial cell morphology, and enhanced expression of p63, p40, ABCB5, and PAX6 at protein level.

### General features of culture conditions and media

Any culture medium may be considered to consist of basal medium and supplements. In the present induction medium, the two essential supplements are TGF-beta inhibitor, and a fibroblast growth factor, whereas in the present corneal epithelial differentiation and maturation medium, exemplary or preferred supplements selected from the group consisting of EGF, hydrocortisone, insulin, isoproterenol, and tri-iodo-thyronine. However, in the context of culture media, further supplements common in the art may be applied, unless they are known to direct differentiation towards tissues other than the eye. When referring to components of a medium, the term includes both supplements and ingredients to the basal medium.

When in use or when ready for use, the present culture media comprise appropriate essential supplements set forth above. However, according to common practice in the field, the ingredients for a medium may be provided as a concentrate comprising said components or a set of vials from which suitable combination is prepared prior to use in a laboratory according to instructions provided. Often, culture medium is diluted and prepared to the final composition immediately before use. Therefore, it is understood that any stock solution or preparation kit suitable for use in such immediate preparation may be used for obtaining cell culture media to be used in the present method. For example, for the preparation of an induction medium set forth above, a cell culture kit comprising the TGF-beta inhibitor and the fibroblast growth factor, each in a separate container or in any combinations thereof, as supplements, and optionally other components, such as basal medium or supplies for preparation thereof, may be employed.

As referred here, "culture medium" or "cultivation medium" refers broadly to any liquid or gel formulation designed to support the growth of microorganisms, cells or small plants. When referring to formulation designed for cell maintenance and growth, term "cell culture medium" is used. In the art, expressions such as induction medium, growth medium, differentiation medium, maturation medium etc. can be considered as subspecies to the general expression "culture medium". A man skilled in the art is familiar with the basic components necessary to maintain and nourish living cells in or on the culture medium, and commercial basic media are widely available. Typically such basic components are referred to as "basal medium", which contains necessary amino acids, minerals, vitamins and organic compounds. Generally, a basal medium may be combined from isolated and pure ingredients. If desired, basal medium may be supplemented with substances contributing to special features or functions of culture medium. Very common supplements include antibiotics, which are used to limit growth of contaminants, L-glutamate and serum, serum albumin or a serum replacement. Those skilled in the art are familiar with both necessary or optional culture medium component and concentrations thereof.

For use in the present method and its various embodiments, the basal medium may be any stem cell culture medium in which stem cells can effectively be differentiated. Non-limiting examples of suitable basal media include KnockOut Dulbecco's Modified Eagle's Medium (KO-DMEM), Dulbecco's Modified Eagle's Medium (DMEM), Minimal Essential Medium (MEM), Basal Medium Eagle (BME), RPMI 1640, F-10, F-12, Glasgow's Minimal Essential Medium (G-MEM), Iscove's Modified Dulbecco's Medium and any combinations thereof. In some preferred embodiments, RegES medium altered by omitting retinol, bFGF and activin A (referred to herein as RegESbasic and disclosed in Vaajasaari et al., Mol Vis. 2011; 17:558-75) is used as a basal medium. In some more preferred embodiments, RegESbasic is used as a basal medium in the present induction medium.

For better clinical acceptance, all culture media to be used in the present method and its various embodiments are preferably substantially xeno-free, substantially serum-free, or substantially defined, more preferably combinations of these, and most preferably substantially xeno-free, substantially serum-free, and substantially defined at the same time. With "substantially" is meant herein that unintentional traces are irrelevant, and what is under clinical or laboratory regulations considered and accepted as xeno-free, serum-free or defined, applies here as well.

As used herein the term "xeno-free" refers to absence of any foreign material or components. Thus, in case of human cell culture, this refers to conditions free from non-human animal components. In other words, when xeno-free conditions are desired for production of corneal cells for human use, all components of any cell culture media must be of human or recombinant origin.

Traditionally, serum, especially fetal bovine serum (FBS) has been valued in cell cultures providing essential growth and survival components for *in vitro* cell culture of eukaryotic cells. It is produced from blood collected at commercial slaughterhouses from cattle bred to supply meat destined for human consumption. "Serum free" indicates that the culture medium contains no serum, either animal or human. Defined medium is valuated when there are contradictions for use of undefined media, e.g. "conditioned medium", which refers to spent media harvested from cultured cells containing metabolites, growth factors, and extracellular matrix proteins secreted into the medium by the cultured cells. Undefined media may be subject to considerable dissimilarities due to natural variation in biology. Undefined components in a cell culture compromise the repeatability of cell model experiments e.g. in drug discovery and toxicology studies. Hence, "defined medium" or "defined culture medium" refers to a composition, wherein the medium has known quantities of all ingredients. Typically, serum that would normally be added to culture medium for cell culture is replaced by known quantities of serum components, such as, e.g., albumin, insulin, transferrin and possibly specific growth factors (e.g.., basic fibroblast growth factor, transforming growth factor or platelet-derived growth factor).

A chemically defined medium is a growth medium suitable for *in vitro* cell culture of human or animal cells in which all of the chemical components are known. A chemically defined medium is entirely free of animal-derived components and represents the purest and most consistent cell culture environment. By definition chemically defined media cannot contain fetal bovine serum, bovine serum albumin or human serum albumin as these products are derived from bovine or human sources and contain complex mixes of albumins and lipids.

Chemically defined media differ from serum-free media in that bovine serum albumin or human serum albumin is replaced with either a chemically defined recombinant version (which lacks the albumin associated lipids) or a synthetic chemical, such as the polymer polyvinyl alcohol, which can reproduce some of the functions of BSA/HSA. The next level of defined media, below chemically defined media is protein-free media. These media contain animal protein hydrolysates and are complex to formulate although are commonly used for insect or CHO cell culture.

According to some embodiments, the present media comprises a xeno-free serum replacement formulation. A defined xeno-free serum replacement formulation or composition may be used to supplement any suitable basal medium for use in *in vitro* derivation, maintenance, proliferation, or differentiation of stem cells. Said serum replacement may be used to supplement either serum-free or serum-containing basal mediums, or any combinations thereof. When xeno-free basal medium is supplemented with a xeno-free serum replacement, the final culture medium is xeno-free as well. One example is described in Rajala et al. 2010, which is incorporated here as reference, describing a xeno-free serum replacement applicable in the context of the present invention. Another non-limiting example of a serum replacement is KnockOut^{™} Serum Replacement (Ko-SR), and xeno-free version KnockOut^{™} SR XenoFree CTS^{™} both commercially available from Life Technologies.

### EXPERIMENTAL PART

### Comparative Example 1

The current standard human pluripotent stem cell culture was performed on human foreskin feeder cells (hFF, CRL-2429, American Type Culture Collection, ATCC, Manassas, USA). The feeder cells were cultured using 10% fetal bovine serum (FBS). The hFF cells were inactivated with f.ex. Mitomycin C treatment (10 µg/ml, 3 hours at, +37°C). Pluripotent stem cells were plated on top of confluent feeder cells in pluripotent stem cell culture medium supplemented with 20% Knockout Serum Replacement (ko-SR) that contained animal-derived products such as bovine serum albumin (BSA). Xeno-Free alternatives such as RegES medium (Rajala etal., PLoS One. 2010; 5(4): e10246) can be used instead as hPSC culture medium. As human pluripotent stem cells were cultured on feeder cells they grew as typical multicellular colonies. To prevent spontaneous differentiation the cell culture medium was changed five times a week and undifferentiated parts of pluripotent stem cell colonies were manually cut with scalpel and transferred to freshly made feeder layers once a week. Alternatively the cells could be passaged to a fresh feeder layer as single cells with TrypLE^{™} Select Enzyme (Life Technologies) every 10 days.

Feeder-free culture, xeno-free culture of human pluripotent stem cells was based on the use of recombinant Laminin-521 (Biolamina, Sweden) extracellular matrix protein coating and defined, xeno-free culture medium, the Essential 8^{™} Flex Medium (Life Technologies). The pluripotent stem cells were transferred to the culture system from the standard feeder cell based culture system by manually cutting undifferentiated colony parts with scalpel and plating to ln-521 coated (0.75-1.8 µg/cm²overnight at +4°C) culture plates. The cells are allowed to grow near confluency with medium changed 3-4 times a week, and passaged with TrypLE^{™} Select Enzyme as single cells twice a week.

Human pluripotent stem cells showed undifferentiated colony morphology and marker expression (OCT-3/4, Nanog, SSEA-3, SSEA-4, TRA-1-60, TRA-1-81) in both feeder dependent and feeder free culture conditions. As quantified with flowcytometry, human pluripotent stem cells cultured in feeder free culture conditions were 99% positive for both SSEA-4 and OCT-3/4 while those cultured on feeder cells were 92% positive for SSEA-4 and 85% positive for OCT-3/4.

Differentiation of pluripotent stem cells cultured on feeder cells to corneal epithelial cells with the induction method disclosed in EP 2828380 comprising of TGF-beta inhibitor, Wnt inhibitor and a fibroblast growth factor (10 µM SB-505124 + 10 µM IWP-2, 50 ng/ml bFGF), followed by differentiation in Cnt-30 epithelial medium on collagen IV matrix, yielded at best >90% p63 positive cells with correct corneal epithelial morphology. While differentiation of pluripotent cells cultured in feeder free conditions with the induction method disclosed in EP 2828380 (with the addition of EB formation with 5µM Blebbistatin overnight) resulted in massive cell death and loss of cells within 2 weeks.

Changing the matrix used for adherent culture from 5 µg/cm² collagen IV to a combination matrix of 5 µg/cm² collagen IV and 0.75 µg/cm² laminin-521 allowed for better cell attachment and survival. However, prominent neuronal differentiation and cell detachment was recurrently observed as the induction method disclosed in EP 2828380 was used. Omitting the Wnt inhibitor IWP-2 from the first phase induction medium notably enhanced cell survival. Additional induction with BMP-4 (induction phase 3), led to best result with most cells having correct epithelial morphology and marker expression and less cells differentiating to other cells types after total of 20-30 days of differentiation.

### Example 2

The human pluripotent stem cells cultured in the feeder free culture system as described above were differentiated to corneal epithelial cells by detaching the pluripotent cells with TrypLE^{™} Select Enzyme for 4 min at +37°C. On Day 0, embryoid bodies were created with overnight incubation with 5µM Blebbistatin (Sigma, B05601) or 10µM Y-27632 dihydrochloride (R&D Systems, 1254) in a xeno-free culture medium containing: Knockout-Dulbecco's Modified Eagle's Medium (ko-DMEM) supplemented with 15% Xeno-Free Knockout Serum Replacement (XF-ko-SR), 2 mM GlutaMax (all from ThermoFisher Scienfic), 1% MEM Eagle Non-Essential Amino acid solution (NEAA, Cambrex Bio Science), 50 U/ml penicillin/streptomycin (Cambrex Bio Science), 0.1 mM β-mercaptoethanol (ThermoFisher Scienfic).

The following day (Day 1) the medium was changed to induction medium: xeno-free culture medium supplemented with 10 µM SB-505124 and 50 ng/ml bFGF, and cells were incubated in this medium overnight. The purpose of this treatment is to push the cells toward ectodermal differentiation pathway instead of mesodermal or endodermal pathways.

During the following two days (Day 2 and Day 3) the medium was changed to xeno-free culture medium supplemented with 25 ng/ml BMP-4 (Peprotech). The purpose of this treatment is to push the cells toward surface ectoderm differentiation and eye field instead of neuronal fate. Without the BMP-4 induction, the cells showed tendency to differentiation toward neuronal fate and/or were lost due to detachment. Adding BMP-4 induction for 2 to 3 days after initial induction led to best result of most cells with correct corneal epithelial cell morphology and marker expression.

After the BMP-4 induction (Day 4), the cells were plated down to 5 µg/cm² collagen IV and 0.75 µg/cm² laminin-521 coated 12-well plates (Corning Cellbind) in Cnt-30 medium. The culture medium was changed three times a week.

It will be obvious to a person skilled in the art that, as the technology advances, the inventive concept can be implemented in various ways. The invention and its embodiments are not limited to the examples described above but may vary within the scope of the claims.

## Claims

1. A method of producing differentiated eye cells selected from the group consisting of corneal epithelial precursor cells, corneal epithelial cells and stratified corneal epithelium, the method comprising:
a) culturing pluripotent stem cells in the absence of feeder cells;
b) culturing said cells in a cell culture medium comprising a TGF-beta inhibitor and a fibroblast growth factor (FGF) followed by culturing said cells in a cell culture medium comprising bone morphogenetic protein 4 (BMP-4) thereby producing eye precursor cells;
c) culturing on a substrate coated at least with collagen IV and laminin said eye precursor cells in a cell culture medium comprising one or more supplements selected from the group consisting of epidermal growth factor (EGF), hydrocortisone, insulin, isoproterenol, and tri-iodo-thyronine in the absence of a TGF-beta inhibitor, FGF, or BMP-4, thereby producing corneal epithelial precursor cells; and
d) optionally, maturating said corneal epithelial precursor cells further into mature corneal epithelial cells or into corneal stratified epithelium;
wherein the culture medium used in b) and c) does not comprise a Wnt-inhibitor.

2. The method according to claim 1, wherein the TGF-beta inhibitor is selected from TGF-beta inhibitors having molar mass of less than 800 g/mol, preferably less than 500 g/mol.

3. The method according to claim 2, wherein the TGF-beta inhibitor is selected from organic molecules according to Formula I 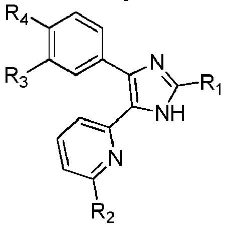 wherein R₁ represents an C₁-C₅ aliphatic alkyl group, carboxylic acid, amide, and R₂ represents an C₁-C₅ aliphatic alkyl, R₃ and R₄ represent aliphatic alkyls including heteroatoms, 0 or N, which may be linked together to form a 5- or 6-member heteroring.

4. The method according to any one of the preceding claims, wherein fibroblast growth factor is selected from basic FGF.

5. The method according to any one of the preceding claims, wherein the amount of TGF-beta inhibitor is from 1 µM to 100 µM, preferably from 1 to 30 µM.

6. The method according to any one of the preceding claims, wherein the amount of fibroblast growth factor is from 1 ng/ml to about 1000 ng/ml, preferably about 2 ng/ml to about 100 ng/ml, and more preferably about 30 ng/ml to about 80 ng/ml.

7. The method according to any one of the preceding claims, wherein the amount of BMP-4 is 1 ng/ml to 1000 ng/ml, preferably about 10 ng/ml to 50 ng/ml, and more preferably 25 ng/ml.

8. The method according to any one of the preceding claims, wherein the stem cells are selected from induced pluripotent stem (iPS) cells and embryonic stem (ES) cells, with the proviso that if human embryonic stem (hES) cells are used, the method does not include the destruction of human embryos.

9. The method according to any one of the preceding claims, wherein step a) comprises forming embryoid bodies from said pluripotent stem cells.

10. The method according to claim 9, wherein said forming of embryoid bodies in step a) is carried out by a physical or chemical method, preferably selected from the group consisting of culturing cells in the presence of attachment-preventing agents, culturing cells in hanging drops, microfabrication techniques, forced aggregation e.g. by centrifugation, and culturing cells in the presence of one or more aggregation-promoting agents such as macromolecular crowders, blebbistatin and ROCK inhibitors.

11. The method according to any one of the preceding claims, wherein the laminin is laminin-521 and/or laminin-511.

12. The method according to any one of the preceding claims, wherein at least 65%, preferably at least 75%, most preferably at least 90% of the corneal epithelial precursor cells express marker p63, based on the total cell population obtained.

13. The method according to any one of the preceding claims, which is performed in substantially xeno-free, substantially serum-free and/or defined conditions.

## Patentansprüche

1. Verfahren zur Herstellung von differenzierten Augenzellen, ausgewählt aus der Gruppe bestehend aus Hornhautepithel-Vorläuferzellen, Hornhautepithelzellen und geschichtetem Hornhautepithel, wobei das Verfahren umfasst:
a) Kultivierung pluripotenter Stammzellen in Abwesenheit von Nährzellen;
b) Kultivieren der Zellen in einem Zellkulturmedium enthaltend einen TGF-beta-Inhibitor und einen Fibroblasten-Wachstumsfaktor (FGF), gefolgt von Kultivieren der Zellen in einem Zellkulturmedium enthaltend morphogenetisches Knochenprotein 4 (BMP-4), wodurch Augenvorläuferzellen erzeugt werden;
c) Kultivierung der Augenvorläuferzellen auf einem mindestens mit Kollagen IV und Laminin beschichteten Substrat in einem Zellkulturmedium enthaltend ein oder mehrere Zusätze ausgewählt aus der Gruppe bestehend aus epidermalem Wachstumsfaktor (EGF), Hydrocortison, Insulin, Isoproterenol und Trijodthyronin, in Abwesenheit eines TGF-beta-Inhibitors, FGF oder BMP-4, wodurch Vorläuferzellen des Hornhautepithels erzeugt werden; und
d) gegebenenfalls weitere Reifung der Vorläuferzellen des Hornhautepithels zu reifen Hornhautepithelzellen oder zu geschichtetem Hornhautepithel;
wobei das in b) und c) verwendete Kulturmedium keinen Wnt-Inhibitor enthält.

2. Verfahren nach Anspruch 1, wobei der TGF-beta-Inhibitor ausgewählt ist aus TGF-beta-Inhibitoren mit einer Molmasse von weniger als 800 g/mol, vorzugsweise weniger als 500 g/mol.

3. Verfahren nach Anspruch 2, wobei der TGF-beta-Inhibitor aus organischen Molekülen gemäß Formel I 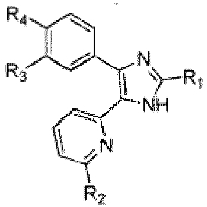 ausgewählt ist,
worin R₁ einer aliphatischen C₁-C₅-Alkylgruppe, einer Carbonsäure, einem Amid entspricht und R₂ einer aliphatischen C₁-C₅-Alkylgruppe entspricht, R₃ und R₄ aliphatischen Alkylgruppen mit Heteroatomen, O oder N, die miteinander verbunden sein können, um einen 5- oder 6-gliedrigen Heteroring zu bilden, entspricht.

4. Verfahren nach einem der vorhergehenden Ansprüche, wobei der Fibroblasten-Wachstumsfaktor ausgewählt ist aus basischem FGF.

5. Verfahren nach einem der vorhergehenden Ansprüche, wobei die Menge des TGF-beta-Inhibitors 1 µM bis 100 µM, vorzugsweise 1 bis 30 µM, beträgt.

6. Verfahren nach einem der vorhergehenden Ansprüche, wobei die Menge des Fibroblasten-Wachstumsfaktors 1 ng/ml bis etwa 1000 ng/ml, vorzugsweise etwa 2 ng/ml bis etwa 100 ng/ml und noch bevorzugter etwa 30 ng/ml bis etwa 80 ng/ml beträgt.

7. Verfahren nach einem der vorhergehenden Ansprüche, wobei die Menge an BMP-4 1 ng/ml bis 1000 ng/ml, vorzugsweise etwa 10 ng/ml bis 50 ng/ml und besonders bevorzugt 25 ng/ml beträgt.

8. Verfahren nach einem der vorhergehenden Ansprüche, wobei die Stammzellen aus induzierten pluripotenten Stammzellen (iPS-Zellen) und embryonalen Stammzellen (ES-Zellen) ausgewählt werden, mit der Maßgabe, dass, wenn menschliche embryonale Stammzellen (hES-Zellen) verwendet werden, das Verfahren nicht die Zerstörung menschlicher Embryonen umfasst.

9. Verfahren nach einem der vorangehenden Ansprüche, wobei Schritt a) die Bildung von Embryoid Bodies aus den pluripotenten Stammzellen umfasst.

10. Verfahren nach Anspruch 9, wobei die Bildung von Embryoid Bodies in Schritt a) durch ein physikalisches oder chemisches Verfahren durchgeführt wird, das vorzugsweise aus der Gruppe bestehend aus der Kultivierung von Zellen in Gegenwart von Substanzen zur Verhinderung von Anhaftungen, der Kultivierung von Zellen in hängenden Tropfen, Mikrofabrikationstechniken, erzwungener Aggregation, z.B. durch Zentrifugation, und der Kultivierung von Zellen in Gegenwart eines oder mehrerer aggregationsfördernder Mittel, wie makromolekularer Crowdern, Blebbistatin und ROCK-Inhibitoren ausgewählt ist.

11. Verfahren nach einem der vorhergehenden Ansprüche, wobei das Laminin Laminin-521 und/oder Laminin-511 ist.

12. Verfahren nach einem der vorangehenden Ansprüche, wobei mindestens 65 %, vorzugsweise mindestens 75 %, am meisten bevorzugt mindestens 90 % der Vorläuferzellen des Hornhautepithels den Marker p63 exprimieren, bezogen auf die gesamte erhaltene Zellpopulation.

13. Verfahren nach einem der vorangehenden Ansprüche, das unter im wesentlichen xenofreien, im wesentlichen serumfreien und/oder definierten Bedingungen durchgeführt wird.

## Revendications

1. Procédé de production de cellules oculaires différenciées choisies dans le groupe constitué par des cellules précurseurs épithéliales cornéennes, des cellules épithéliales cornéennes et l'épithélium cornéen stratifié, le procédé comprenant :
a) la mise en culture de cellules souches pluripotentes en l'absence de cellules nourricières ;
b) la mise en culture desdites cellules dans un milieu de culture cellulaire comprenant un inhibiteur de TGF-bêta et un facteur de croissance des fibroblastes (FGF) suivie de la mise en culture desdites cellules dans un milieu de culture cellulaire comprenant la protéine morphogénétique osseuse 4 (BMP-4] produisant ainsi des cellules précurseurs oculaires ;
c) la mise en culture, sur un substrat revêtu au moins de collagène IV et de laminine, desdites cellules précurseurs oculaires dans un milieu de culture cellulaire comprenant un ou plusieurs compléments choisis dans le groupe constitué par le facteur de croissance épidermique (EGF), l'hydrocortisone, l'insuline, l'isoprotérénol et la triiodothyronine en l'absence d'un inhibiteur de TGF-bêta, de FGF ou de BMP-4, produisant ainsi des cellules précurseurs épithéliales cornéennes ; et
d) éventuellement, la maturation ultérieure desdites cellules précurseurs épithéliales cornéennes en cellules épithéliales cornéennes matures ou en épithélium cornéen stratifié ;
dans lequel le milieu de culture utilisé dans b) et c) ne comprend pas d'inhibiteur de Wnt.

2. Procédé selon la revendication 1, dans lequel l'inhibiteur de TGF-bêta est choisi parmi des inhibiteurs de TGF-bêta ayant une masse molaire inférieure à 800 g/mol, de préférence inférieure à 500 g/mol.

3. Procédé selon la revendication 2, dans lequel l'inhibiteur de TGF-bêta est choisi parmi des molécules organiques selon la Formule 1 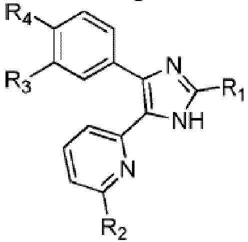 dans laquelle R₁ représente un groupe alkyle aliphatique en C₁ à C₅, un acide carboxylique, un amide, et R₂ représente un alkyle aliphatique en C₁ à C₅, R₃ et R₄ représentent des alkyles aliphatiques comprenant des hétéroatomes, O ou N, qui peuvent être liés ensemble pour former un hétérocycle penta- ou hexagonal.

4. Procédé selon l'une quelconque des revendications précédentes, dans lequel le facteur de croissance des fibroblastes est choisi parmi un FGF basique.

5. Procédé selon l'une quelconque des revendications précédentes, dans lequel la quantité d'inhibiteur de TGF-bêta est comprise entre 1 µM et 100 µM, de préférence entre 1 et 30 µM.

6. Procédé selon l'une quelconque des revendications précédentes, dans lequel la quantité de facteur de croissance des fibroblastes est comprise entre 1 ng/ml et environ 1000 ng/ml, de préférence entre environ 2 ng/ml et environ 100 ng/ml, et plus préférablement entre environ 30 ng/ml et environ 80 ng/ml.

7. Procédé selon l'une quelconque des revendications précédentes, dans lequel la quantité de BMP-4 est comprise entre 1 ng/ml et 1000 ng/ml, de préférence entre environ 10 ng/ml et 50 ng/ml, et plus préférablement de 25 ng/ml.

8. Procédé selon l'une quelconque des revendications précédentes, dans lequel les cellules souches sont choisies parmi des cellules souches pluripotentes induites (iPS) et des cellules souches embryonnaires (ES), à condition que si des cellules souches embryonnaires humaines (hES) sont utilisées, le procédé ne comprenne pas la destruction d'embryons humains.

9. Procédé selon l'une quelconque des revendications précédentes, dans lequel l'étape a) comprend la formation de corps embryoïdes à partir desdites cellules souches pluripotentes.

10. Procédé selon la revendication 9, dans lequel ladite formation de corps embryoïdes à l'étape a) est réalisée par un procédé physique ou chimique, de préférence choisi dans le groupe constitué par la mise en culture de cellules en présence d'agents empêchant la fixation, la mise en culture de cellules en gouttes suspendues, des techniques de microfabrication, l'agrégation forcée par exemple par centrifugation, et la mise en culture de cellules en présence d'un ou de plusieurs agents favorisant l'agrégation tels que des agents d'encombrement macromoléculaire, la blebbistatine et des inhibiteurs de ROCK.

11. Procédé selon l'une quelconque des revendications précédentes, dans lequel la laminine est la laminine-521 et/ou la laminine-511.

12. Procédé selon l'une quelconque des revendications précédentes, dans lequel au moins 65%, de préférence au moins 75%, idéalement au moins 90% des cellules précurseurs épithéliales cornéennes expriment le marqueur p63, par rapport à la population cellulaire totale obtenue.

13. Procédé selon l'une quelconque des revendications précédentes, qui est réalisé dans des conditions sensiblement exemptes de xéno-contaminants, sensiblement exemptes de sérum et/ou définies.
